(19) Europäisches Patentamt
European Patent Office
Office européen des brevets

(11) Veröffentlichungsnummer: **0 042 592**
**B1**

(12) # EUROPÄISCHE PATENTSCHRIFT

(45) Veröffentlichungstag der Patentschrift: **30.01.85**

(21) Anmeldenummer: **81104679.6**

(22) Anmeldetag: **19.06.81**

(51) Int. Cl.$^4$: **C 07 D 231/44,**
C 07 D 231/38,
C 07 D 231/56, C 07 D 249/18

(54) **N-Heteroaryl-alkylendiamine, Verfahren zu ihrer Herstellung sowie ihre Verwendung.**

(30) Priorität: **23.06.80 DE 3023369**

(43) Veröffentlichungstag der Anmeldung:
**30.12.81 Patentblatt 81/52**

(45) Bekanntmachung des Hinweises auf die Patenterteilung:
**30.01.85 Patentblatt 85/05**

(84) Benannte Vertragsstaaten:
**AT BE CH DE FR GB IT LI LU NL SE**

(56) Entgegenhaltungen:
**FR-A-2 158 808**
**US-A-4 204 998**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH**
**Patentabteilung, Abt. E Sandhofer Strasse 112-132 Postfach 31 01 20**
**D-6800 Mannheim 31 Waldhof (DE)**

(72) Erfinder: **Wiedemann, Fritz, Dr.**
**Weinheimer Strasse 82**
**D-6940 Weinheim-Lützelsachsen (DE)**
Erfinder: **Rieger, Ewald**
**Seckenheimerlandstrasse 45**
**D-6800 Mannheim-25 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 042 592 B1

**Beschreibung**

Die vorliegende Erfindung betrifft neue N-Heteroarylalkylendiamine der allgemeinen Formel I, welche wertvolle Zwischenprodukte für die Herstellung von Herz- und Kreislauf-wirksamen Substanzen darstellen, sowie Verfahren zu ihrer Herstellung.

Verbindungen ähnlicher Struktur sind in der DE—A—28 19 629 (Uracile), den US—A—4 204 998 und FR—A—2 158 808 (Bentriazole) als Zwischenprodukte beschrieben.

Gegenstand der Erfindung sind N-Heteroaryl-alkylendiamine der allgemeinen Formel I

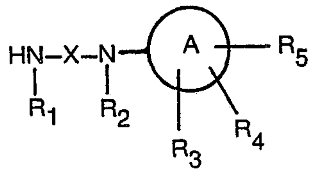
(I)

in welcher

$R_1$, $R_2$ gleich oder verschieden sein können und Wasserstoff, $C_1$—$C_4$-Alkyl oder Benzyl,

X eine geradkettige Ethylen- oder Propylengruppe,

A einen mit einem Ring-Kohlenstoffatom an den Stickstoff gebundenen Pyrazol-, Pyrazolon- oder Indazol-Rest und

$R_3$, $R_4$ und $R_5$ gleich oder verschieden sein können und Wasserstoff, $C_1$—$C_4$-Alkyl oder Allyl bedeuten, sowie deren Salze.

Da Verbindungen der Formel I asymmetrische Kohlenstoffatome besitzen können, sind ferner Gegenstand der Erfindung die optisch aktiven Formen und racemischen Gemische dieser Verbindungen.

Unter den $C_1$—$C_4$-Alkylgruppen in den Resten $R_1$, $R_2$, $R_3$, $R_4$ oder $R_5$ sind geradkettige oder verzweigte Gruppen zu verstehen, wie zum Beispiel Methyl, Ethyl, Isopropyl, Butyl. Besonders bevorzugt ist die Methylgruppe.

Die neuen Verbindungen der Formel I sind geeignete Zwischenprodukte zur Herstellung von Verbindungen mit wertvollen pharmakologischen Eigenschaften, zum Beispiel zur Herstellung von Herz- und Kreislauf-wirksamen Verbindungen, wie sie in der EP—A—42 593 beschrieben und beansprucht sind.

So kann beispielsweise aus 4-(2-Amino-ethylamino)-indazol (Beispiel 3 dieser Anmeldung) durch Umsetzung mit Phenylglycidether das cardioton wirksame 1-Phenoxy-3-[2-(4-indazolylamino)-ethylamino]-propan-2-ol (Beispiel 13 von EP—A—42 593) erhalten werden.

Ein weiter Gegenstand der Erfindung sind Verfahren zur Herstellung von Verbindungen der allgemeinen Formel I.

Die Verbindungen werden erhalten, indem man in an sich bekannter Weise entweder

a) eine Verbindung der allgemeinen Formel II

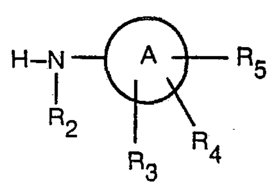
(II)

in welcher $R_2$, A, $R_3$, $R_4$, $R_5$ die angegebenen Bedeutungen haben mit einer Verbindung der allgemeinen Formel III

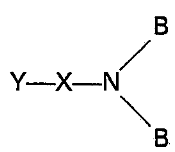
(III)

in welcher X die angegebene Bedeutung hat, Y ein reaktiver Rest und B eine Schutzgruppe darstellt, umsetzt und die Schutzgruppe anschließend entfernt oder

b) eine Verbindung der allgemeinen Formel IV

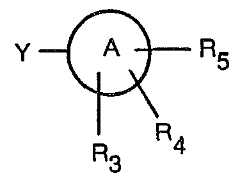

(IV)

in welcher A, $R_3$, $R_4$, $R_5$ die angegebenen Bedeutungen haben und Y ein reaktiver Rest darstellt, mit einer Verbindung der allgemeinen Formel V

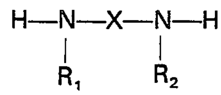

(V)

in welcher X, $R_1$, $R_2$ die angegebenen Bedeutungen haben, umsetzt oder

c) eine Verbindung der allgemeinen Formel IV′

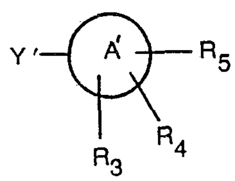

(IV′)

in welcher $R_3$, $R_4$, $R_5$ die angegebenen Bedeutungen haben, A′ einen Indazolrest und Y′ eine Hydroxyl- oder eine Aminogruppe darstellt mit tiner Verbindung der allgemeinen Formel V, in welcher X, $R_1$, $R_2$ die angegebenen Bedeutungen haben und welche in diesem Fall in Form ihrer Salze der schwefligen Säure zur Anwendung kommt (Bucherer-Lepetit-Reaktion) umsetzt, anschließend gegebenenfalls die erhalten Verbindungen der allgemeinen Formel I in andere Verbindungen der Formel I oder mit Säuren in Salze in an sich bekannter Weise überführt und gegebenenfalls ein racemisches Gemisch von Verbindungen der allgemeinen Formel I in an sich bekannter Weise in die optisch aktiven Formen trennt.

Unter reaktiven Resten Y der allgemeinen Formeln III und IV sind z.B. die Reste von Halogenwasserstoffsäuren bzw. Sulfonsäuren, Chloride, Bromide, Mesyloxy-, Tosyloxy-, Methylmercapto- und Methylsulfonyl-gruppen zu verstehen. Bevorzugt sind die Chloride und Bromide.

Als Schutzgruppen B in der allgemeinen Formel III kommen im Prinzip alle üblichen Stickstoffschutzgruppen in Frage. Solche Schutzgruppen sind zum Beispiel Benzylgruppen und die Phthaloylgruppe, welche auch besonders bevorzugt sind und sich durch Hydrogenolyse bzw. Hydrazinolyse entfernen lassen.

Die Umsetzungen nach Variante a koennen in einem inerten Loesungsmittel wie Dimethylformamid oder Acetonitril und Zusatz einer Base durchgeführt werden.

Besonders bevorzugt sind Umsetzungen in Acetonitril in Gegenwart von Kaliumcarbonat bei 80°C.

Bei Umsetzungen nach Variante b ist ein großer Überschuss der Aminkomponente V vorteilhaft. Man arbeitet in einem inerten Lösungsmittel wie Dimethylsulfoxid oder ohne Lösungsmittel bei Temperaturen zwischen 20 und 120°C, beispielsweise in Ethylendiamin bei 118°C.

Auch bei Variante c ist ein großer Überschuss des Alkylendiamins V vorteilhaft, welches in Form seiner Salze der schwefligen Säure zur Umsetzung kommt.

Man arbeitet vorzugsweise in Wasser oder in einem Gemisch aus Wasser und einem Alkohol wie Ethylenglycol oder n-Propanol bei Rückflußtemperatur.

Die Verbindungen der Formel I können nach den Herstellungsverfahren direkt als Salze erhalten werden oder gewünschtenfalls nach an sich bekannten Verfahren in Salze überführt werden. Als Salze kommen insbesondere die Umsetzungsprodukte mit Halogenwasserstoffsäure, wie Salzsäure, oder Oxalsäure in Frage.

Neben den in den folgenden Beispielen aufgeführten Verbindungen sind auch bevorzugt:

4-(2-Amino-ethylamino)-3.5 -dimethyl-pyrazol
4-(3-Amino-propylamino)-1.3.5-trimethyl-pyrazol
4-(2-Amino-ethylamino)-1-allyl-3.5-dimethyl-pyrazol
4-(2-Amino-ethylamino)-1-ethyl-3.5-dimethyl-pyrazol
6-(2-Amino-ethylamino)-indazol
4-(2-Amino-ethylamino)-2-methyl-indazol

Die Erfindung wird durch die folgenden Beispiele näher erläutert:

Beispiel 1

4-(2-Amino-ethylamino)-1.3.5-trimethyl-pyrazol

39.1 g 4-(2-Phthalimido-ethylamino)-1.3.5-trimethylpyrazol werden mit 7 ml Hydrazinhydrat in 450 ml Ethanol 1 Stunde unter Rückfluß zum Sieden erhitzt. Man säuert durch Zugabe von 6 N Salzsäure an, kocht eine weitere Stunde und kühlt auf 10°C. Nach Absaugen des gebildeten Phthylhydrazids engt man ein, löst den Rückstand in Methanol und entsalzt mit Amberlite® IRA 400 (OH—Form).

Ausbeute: 22.4 g (100% d.Th.) Öl

Die verwendete Ausgangsverbindung

4-(2-Phthalimido-ethylamino)-1.3.5-trimethyl-pyrazol

wird in guter Ausbeute durch Umsetzung von 4-Amino-1.3.5-trimethyl-pyrazol mit N-(2-Bromethyl)-phthalimid erhalten (in Gegenwart von Kaliumcarbonat in Acetonitril 16 Stunden Rückfluß): gelbliche Kristalle, Fp. 122—123°C (Ethanol).

Beispiel 2

3-(2-Amino-ethylamino)-1.2.4-trimethyl-5-pyrazolon

24.7 g 3-Chlor-1.2.4-trimethyl-5-pyrazolon werden in 157 ml Ethylendiamin 22 Stunden unter Rückfluß zum Sieden erhitzt. Man engt ein, löst den Rückstand in Methanol und entsalzt durch den Ionenaustauscher Amberlite® IRA-400 (OH—Form). Das Eluat wird eingeengt, der Rückstand in Methylenchlorid aufgenommen, worauf man über eine Kieselgelsäule mit dem Laufmittel Methylenchlorid-Methanol ($NH_3$-gesättigt) 8:2 reinigt.

Ausbeute: 23 g (81%, d.Th.) gelbliches Öl.

Die verwendete Ausgangsverbindung kann auf folgendem Wege erhalten werden:

3-Chlor-1.2.4-trimethyl-5-pyrazolon

28.6 g 3-Hydroxy-1.2.4-trimethyl-5-pyrazolon (Fp. 87—89°C/Essigester), aus Methylmalonsäurediethylester und N.N'-Dimethylhydrazin hergestellt, werden mit 55 ml Phosphoroxitrichlorid 3.5 Stunden unter Rückfluß zum Sieden erhitzt. Man engt ein, rührt den Rückstand mit Eis + Wasser, stellt durch Zugabe von 10 N Natronlauge alkalisch und extrahiert Methylenchlorid (8 × 100 ml). Der Extrakt wird getrocknet und eingeengt.

Ausbeute: 24.7 g (76.5% d.Th.) farblose Kristalle, Fp. 37—38°C.

Beispiel 3

4-(2-Amino-ethylamino)-indazol

In eine Mischung aus 66 ml Ethylendiamin und 146 ml Wasser wird Schwefeldioxid bis pH 7—8 eingeleitet, worauf man 13.4 g 4-Hydroxyindazol zusetzt und unter Rühren 1 Stunde auf 100—110°C erwärmt. Nach Erkalten versetzt man mit 300 ml Methanol und saugt die gebildeten Salze ab, welche mit Methanol nachgewaschen werden. Man engt schonend im Vakuum auf 50—100 ml ein und sauft das gebildete Nebenprodukt (N,N'-Bis-(indazolyl-4)-ethyldiamin) ab. Danach stellt man mit konz. Ammoniak alkalisch und extrahiert (10×) mit Methylenchlorid, trocknet den Extrakt und engt ihn auf ca. 100 ml ein.

Ausbeute: 7.6 g (43% d.Th.) hellbeige Kristalle, Fp. 138—140°C.

Benzoat: farblose Kristalle, Fp. 178—180°C(Z.), (aus Isopropanol).

Beispiel 4

4-(3-Amino-propylamino)-indazol

In einer Lösung von 74 g 1.3-Diamino-propan in 150 ml Wasser leitet man Schwefeldioxid bis pH 7 ein, worauf man 13.4 g 4-Hydroxy-indazol zugibt und 3 Stunden auf 100°C erhitzt. Durch Zusatz der doppelten Menge Methanol werden Salze ausgefällt, von denen man absaugt. Das Filtrat wird eingeengt, der ölige Rückstand durch konz. Ammoniak alkalisch gestellt, worauf man mit Methylenchlorid extrahiert. Durch Einengen des Extraktes werden 7.8 g (41% d.Th.) graue Kristalle, Fp. 154—163°C erhalten.

Beispiel 5

5-Methyl-4-(2-amino-ethylamino)-indazol

Ein Gemisch aus 15.3 g 4-Amino-5-methyl-indazol (Fp. 197—200°C, durch Reduktion von 4-Nitro-5-methyl-indazol hergestellt), 146 g Ethylendiaminsulfit, 146 ml Ethylenglycol und 146 ml Wasser wird 24 Stunden bei 110°C gerührt.

Nach Erkalten versetzt man mit 500 ml Methanol und saugt die gefällten Salze ab, welche mit Methanol nachgewaschen werden. Das Filtrat läßt man durch den Ionenaustauscher Amberlite® 120 ($H^+$—Form) laufen, wäscht mit Methanol nach und setzt das Amin mit methanolischem Ammoniak frei. Das Eluat wird eingeengt, der Rückstand in Methylenchlorid-Methanol 9:1 gelöst.

Man chromatographiert an einer Kieselgelsäule mit dem Laufmittel Methylenchlorid-Methanol 9:1.

Ausbeute 6.1 g (31% d.Th.) beige Kristalle, Fp. 153—155°C.

Beispiel 6

5-(2-Amino-ethylamino)-indazol

Ein Gemisch aus 12.0 g 5-Amino-indazol, 146 g Ethylendiaminsulfit, 146 ml Wasser und 10 ml n-Propanol wird 24 Stunden unter Rühren auf 100°C erwärmt. Nach Erkalten wird mit 500 ml Methanol verdünnt. Nach Absaugen der ausgefallenen Salze wird das Filtrat eingeengt, der Rückstand durch Zugabe von konz. Ammoniak alkalisch gestellt. Man saugt ab, wäscht mit Wasser nach und erhält 9.4 g (59% d.Th.) Kristalle, Fp. 150—165°C.

Beispiel 7

7-(2-Amino-ethylamino)-indazol

13.4 g 7-Hydroxy-indazol werden mit 146 g Ethylendiaminsulfit in 146 ml Wasser unter Rühren 2—3 Stunden auf 110°C erwärmt. Man saugt noch heiß von gebildetem Nebenprodukt (N,N'-Bis-indazolyl-(7)-ethylendiamin) ab, wäscht mit wenig Wasser nach und fällt durch Zusatz der 5-fachen Menge Methanol die Salze aus, wäscht mit Methanol nach und engt das Filtrat bis zu einer dickflüssigen Konsistenz ein, stellt durch Zugabe von konz. Ammoniak alkalisch, kühlt (Eis), saugt ab und wäscht mit wenig Wasser.

Ausbeute: 11.8 g hellgelbliche Kristalle, 163°Sintern, Fp. 165—167°C.

Durch Umkristallisation aus Essigester-Isopropanol: farblose Kristalle, Fp. 166—168°C.

**Patentansprüche**

1. N-Heteroaryl-alkylendiamine der allgemeinen Formel I

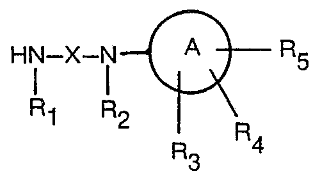

(I)

in welcher

$R_1$, $R_2$ gleich oder verschieden sein können und Wasserstoff, $C_1$—$C_4$-Alkyl oder Benzyl,

X eine geradkettige Ethylen- oder Propylengruppe,

A einen mit einem Ring-Kohlenstoffatom an den Stickstoff gebundenen Pyrazol-, Pyrazolon- oder Indazol-Rest und

$R_3$, $R_4$ und $R_5$ gleich oder verschieden sein können und Wasserstoff, $C_1$—$C_4$-Alkyl oder Allyl bedeuten, sowie deren Salze.

2. 4-(2-Amino-ethylamino)-1.3.5-trimethylpyrazol gemäß Anspruch 1.

3. 4-(2-Amino-ethylamino)-indazol gemäß Anspruch 1.

4. Verfahren zur Herstellung von N-Heteroaryl-alkylendiamine der allgemeinen Formel I

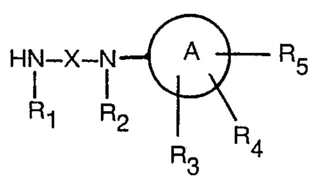

(I)

in welcher

$R_1$, $R_2$ gleich oder verschieden sein können und Wasserstoff, $C_1$—$C_4$-Alkyl oder Benzyl,

X eine geradkettige Ethylen- oder Propylengruppe,

A einen mit einem Ring-Kohlenstoffatom an den Stickstoff gebundenen Pyrazol-, Pyrazolon- oder Indazol-Rest und

$R_3$, $R_4$ und $R_5$ gleich oder verschieden sein können und Wasserstoff, $C_1$—$C_4$-Alkyl oder Allyl bedeuten, sowie deren Salze, dadurch gekennzeichnet, daß man in an sich bekannter Weise

a) eine Verbindung der allgemeinen Formel II

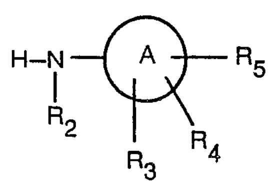

(II)

in welcher $R_2$, A, $R_3$, $R_4$ und $R_5$ die angegebenen Bedeutungen haben mit einer Verbindung der allgemeinen Formel III

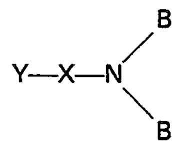

(III)

in welcher X die angegebene Bedeutung hat, Y ein reaktiver Rest und B eine Schutzgruppe darstellt, umsetzt und die Schutzgruppe anschließend entfernt oder

b) eine Verbindung der allgemeinen Formel IV

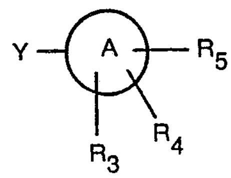

(IV)

in welcher A, $R_3$, $R_4$, $R_5$ die angegebenen Bedeutungen haben und Y ein reaktiver Rest darstellt, mit einer Verbindung der allgemeinen Formel V

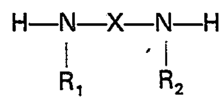

(V)

in welcher X, $R_1$, $R_2$ die angegebenen Bedeutungen haben, umsetzt anschließend gegebenenfalls die erhaltenen Verbindungen der allgemeinen Formel I in andere Verbindungen der Formel I oder mit Säuren in Salze in an sich bekannter Weise überführt.

5. Verfahren nach Anspruch 4 worin A einen mit einem Ring-Kohlenstoffatom gebundenen Indazolrest bedeutet, dadurch gekennzeichnet, daß man eine Verbindung der allgemeinen Formel IV′

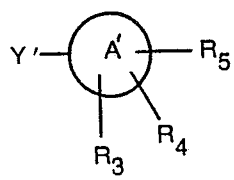

(IV′)

in welcher $R_3$, $R_4$, $R_5$ die angegebenen Bedeutungen haben, A′ einen Indazolrest und Y′ eine Hydroxyl- oder eine Aminogruppe darstellt mit einer Verbindung der allgemeinen Formel V gemäß Anspruch 4, welcher X, $R_1$, $R_2$ die dort angegebenen Bedeutungen haben und welche in diesem Fall in Form ihrer Salze der schwefligen Säure zur Anwendung kommt (Burcherer-Lepetit-Reaktion) umsetzt.

6. Verwendung von Verbindungen gemäß Anspruch 1 als Zwischenprodukte zur Herstellung von pharmakologisch wirksamen Verbindungen der allgemeinen Formel VI

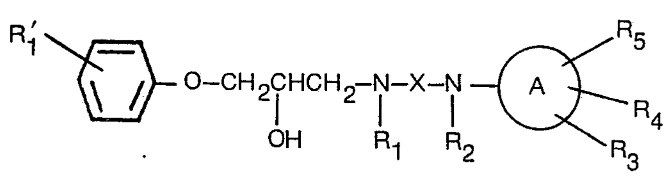

(VI)

in welcher

$R_1'$ ein Wasserstoffatom, $C_1$—$C_4$-Alkyl, Cyan, Carboxamido, Halogen, Hydroxy, $C_1$—$C_5$-Acyloxy, $C_1$—$C_4$-Alkoxy, Allyloxy, Phenyl-$C_1C_4$-alkoxy,

$R_1$ und $R_2$ gleich oder verschieden sind und ein Wasserstoffatom oder $C_1$—$C_4$-Alkyl,

X eine geradkettige Ethylen- oder Propylengruppe.

A einen mit einem Ring-Kohlenstoff an den Stickstoff gebundenen Rest bedeutet, ausgewählt aus der Gruppe Pyrazol, Indazol und Pyrazolon,

$R_3$, $R_4$ und $R_5$ gleich oder verschieden sind und Wasserstoff, $C_1$—$C_4$-Alkyl oder Allyl

bedeuten, deren optisch aktive Formen, racemische Gemische sowie deren pharmakologisch verträgliche Salze.

**Claims**

1. N—Heteroaryl-alkylenediamines of the general formula I

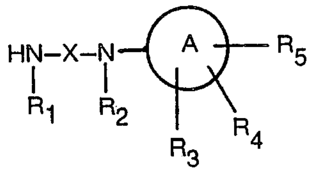

(I)

in which $R_1$, $R_2$ can be the same or different and signify hydrogen, $C_1$—$C_4$-alkyl or benzyl, X signifies a straight-chained ethylene or propylene group, A signifies a pyrazole, pyrazolone or indazole radical connected with a ring carbon atom to the nitrogen and $R_3$, $R_4$ and $R_5$ can be the same or different and signify hydrogen, $C_1$—$C_4$-alkyl or allyl, as well as their salts.

2. 4-(2-Aminoethylamino)-1,3,5-trimethylpyrazole according to claim 1.

3. 4-(2-Aminoethylamino)-indazole according to claim 1.

4. Process for the preparation of N-heteroaryl alkylenediamines of the general formula I

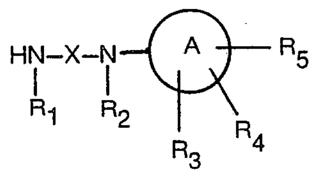

(I)

in which $R_1$, $R_2$ can be the same or different and signify hydrogen, $C_1$—$C_4$-alkyl or benzyl, X signifies a straight-chained ethylene or propylene group, A signifies a pyrazole, pyrazolone or indazole radical connected with a ring carbon atom to the nitrogen and $R_3$, $R_4$ and $R_5$ can be the same or different and signify hydrogen, $C_1$—$C_4$-alkyl or allyl, as well as of their salts, characterised in that, in per se known manner, one

a) reacts a compound of the general formula II

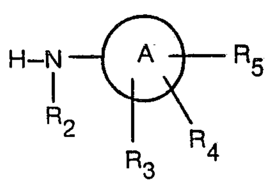

(II)

in which $R_2$, A, $R_3$, $R_4$ and $R_5$ have the given meanings, with a compound of the general formula III

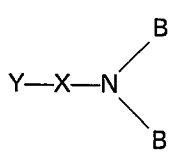

(III)

in which X has the given meaning, Y represents a reactive residue and B represents a protective group, and subsequently removes the protective group or

b) reacts a compound of the general formula IV

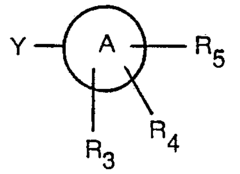

(IV)

in which A, $R_3$, $R_4$, $R_5$ have the given meanings, with a compound of the general formula V

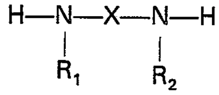

(V)

in which X, $R_1$, $R_2$ have the given meanings, and subsequently optionally converts the compounds obtained of the general formula I into other compounds of the formula I or with acids into salts, in per se known manner.

5. Process according to claim 4, wherein A signifies an indazole radical connected with a ring carbon atom, characterized in that one reacts a compound of the general formula IV′

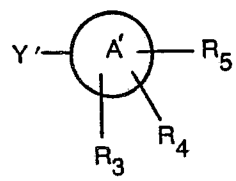

(IV′)

in which $R_3$, $R_4$, $R_5$ have the given meanings, A′ represents an indazole radial and Y′ a hydroxyl or amino group, with a compound of the general formula V according to claim 4, in which X, $R_1$, $R_2$ have the there-given meanings and which, in this case, are used in the form of their salts with sulphurous acid (Bucherer-Lepetit reaction), subsequently optionally converts the compounds obtained of the general formula I into other compounds of the formula I or with acids into salts, in per se known manner.

6. Use of compounds according to claim 1 as intermediate products for the preparation of pharmacologically effective compounds of the general formula VI

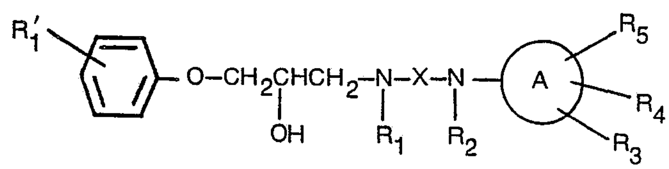

(VI)

in which $R_1'$ signifies a hydrogen atom, $C_1$—$C_4$-alkyl, cyano, carboxamido, halogen, hydroxyl, $C_1$—$C_5$-acyloxy, $C_1$—$C_4$-alkoxy, allyloxy, phenyl-$C_1$—$C_4$-alkoxy, $R_1$ and $R_2$ are the same or different and signify a hydrogen atom or $C_1$—$C_4$-alkyl, X signifies a straight-chained ethylene or propylene group, A a radical selected from the group of pyrazole, indazole and pyrazolone connected with a ring carbon atom to the nitrogen, $R_3$, $R_4$ and $R_5$ are the same or different and signify hydrogen, $C_1$—$C_4$-alkyl or allyl, their optically-active forms, racemic mixtures, as well as their pharmacologically acceptable salts.

**Revendications**

1. N-hétéro-aryl-alkylènediamines de formule générale I:

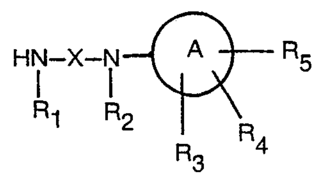

(I)

dans laquelle

$R_1$, $R_2$ peuvent être identiques ou différents et sont de l'hydrogène, de l'alkyle $C_1$—$C_4$ ou du benzyle,

X est un groupe éthylène ou propylène à chaîne droite,

A est un reste pyrazole, pyrazolone ou indazole lié par un atome de carbone du noyau à l'azote et,

$R_3$, $R_4$ et $R_5$ peuvent être identiques ou différents et sont de l'hydrogène, de l'alkyle $C_1$—$C_4$ ou de l'allyle, ainsi que leurs sels.

2. Le 4-(2-amino-éthylamino) 1,3,5-triméthyl-pyrazole selon la revendication 1.

3. Le 4-(2-amino-éthylamino)indazole selon la revendication 1.

4. Procédé de préparation de N-hétéro-aryl-alkylènediaminesde formule générale I

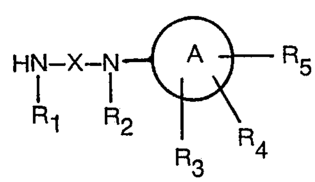

(I)

dans laquelle

$R_1$, $R_2$ peuvent être identiques ou différents et sont de l'hydrogène, de l'alkyle $C_1$—$C_4$ ou du benzyle,

X est un groupe éthylène ou propylène à chaîne droite,

A est un reste pyrazole, pyrazolone ou indazole lié par un atome de carbone du noyau à l'azote et,

$R_3$, $R_4$ et $R_5$ peuvent être identiques ou différents et sont de l'hydrogène, de l'alkyle $C_1$—$C_4$ ou de l'allyle,

ainsi que leurs sels, caractérisé en ce que de façon en soi connue on fait réagir soit:

a) un composé de formule générale II

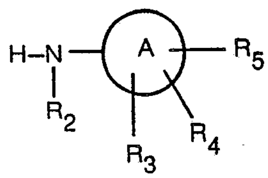

(II)

dans laquelle $R_2$, A, $R_3$, $R_4$ et $R_5$ ont les significations indiquées, avec un composé de formule générale III

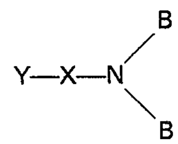

(III)

dans laquelle X a la signification indiquée, Y est un reste actif et B est un groupe de protection, et on élimine ensuite le groupe de protection, ou

b) un composé de formule générale IV

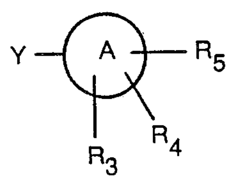

(IV)

dans laquelle A, $R_3$, $R_4$ et $R_5$ ont les significations indiquées et Y est un reste réactif, avec un composé de formule générale V:

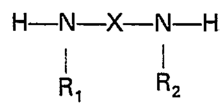

(V)

dans laquelle X, $R_1$ et $R_2$ ont les significations indiquées, et en ce qu'ensuite on transforme éventuellement, de façon et soi connue, les composés obtenus de formule générale I en d'autres composés de formule I ou bien avec des acides en sels.

5. Procédé selon la revendication 4, où A est un reste indazole lié à un atome de carbone du noyau, caractérisé en ce qu'on fait réagir un composé de formule générale IV'

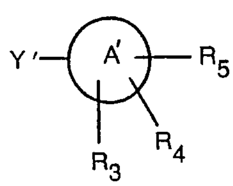

(IV')

dans laquelle $R_3$, $R_4$ et $R_5$ ont les significations indiquées, A' est un reste indazole et Y' un groupe hydroxyle ou amino, avec un composé de formule générale V selon la revendication 4, dans laquelle X, $R_1$ et $R_2$ ont les significations indiquées à cet endroit et qui est mis en oeuvre dans le présent cas sous la forme de ses sels aved l'acide sulfureux (réaction de Bucherer-Lepetit), et en ce qu'ensuite on transforme éventuellement de façon en soi connue les composés obtenus de formule générale I en d'autres composés de formule I ou bien avec des acides en sels.

6. Utilisation de composés selon la revendication 1 en tant que produit intermédiaire pour la préparation de composés pharmacologiquement actifs de formule générale VI.

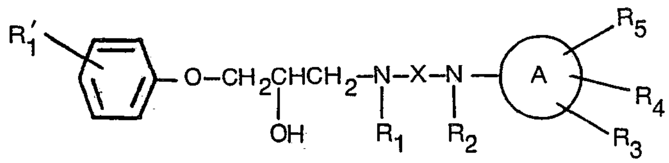

(VI)

dans laquelle

$R_1'$ est un atome d'hydrogène, de l'alkyle $C_1$—$C_4$, du cyano, du carboxamido, de l'halogène, de l'hydroxyle, de l'acyloxy $C_1$—$C_5$, de l'alcoxy $C_1$—$C_4$, de l'allyloxy, du phényl-alcoxy $C_1$—$C_4$,

$R_1$ et $R_2$ sont identiques ou différents et sont un atome d'hydrogène ou de l'alkyle $C_1$—$C_4$,

X est un groupe éthylène ou propylène à chaîne droite,

A est un reste lié par un atome de carbone du noyau à l'azote, choisi parmi le pyrazole, l'indazole et la pyrazolone,

$R_3$, $R_4$ et $R_5$ sont identiques ou différents et sont de l'hydrogène, de l'alkyle $C_1$—$C_4$ ou de l'allyle, leurs formes optiquement actives, leurs mélanges racémiques et leurs sels pharmacologiquement tolérables.